Europäisches Patentamt

European Patent Office

Office européen des brevets

(19)

(11) **EP 0 706 577 B1**

(12) **EUROPEAN PATENT SPECIFICATION**

(45) Date of publication and mention
of the grant of the patent:
**27.08.1997 Bulletin 1997/35**

(21) Application number: **94922002.4**

(22) Date of filing: **29.06.1994**

(51) Int Cl.[6]: **C12P 7/40**, C07F 9/38
// C12P7:40, C12R1:78,
C12P7:40, C12R1:84

(86) International application number:
**PCT/US94/07079**

(87) International publication number:
**WO 95/01449 (12.01.1995 Gazette 1995/03)**

(54) **AN IMPROVED METHOD OF PREPARING GLYOXYLIC ACID/AMINOMETHYLPHOSPHONIC ACID MIXTURES USING A MICROBIAL DOUBLE-TRANSFORMANT**

Verbessertes Verfahren zur Herstellung von Mischuingen aus Glyoxylsäure und Aminoethylphosphonsäure unter Verwendung eines doppelt angereicherten Mikroorganismus

PROCEDE DE PREPARATION AMELIORE DE MELANGES D'ACIDES GLYOXYLIQUE/AMINOMETHYLPHOSPHONIQUE AU MOYEN D'UN DOUBLE TRANSFORMANT MICROBIEN

(84) Designated Contracting States:
**BE ES FR GB IE IT NL**

(30) Priority: **01.07.1993 US 84937**

(43) Date of publication of application:
**17.04.1996 Bulletin 1996/16**

(73) Proprietor: **E.I. DU PONT DE NEMOURS AND COMPANY**
**Wilmington Delaware 19898 (US)**

(72) Inventors:
• **ANTON, David, Leroy**
**Wilmington, DE 19803 (US)**

• **DiCOSIMO, Robert**
**Wilmington, DE 19808 (US)**

(74) Representative: **Hildyard, Edward Martin et al**
**Frank B. Dehn & Co.,**
**European Patent Attorneys,**
**179 Queen Victoria Street**
**London EC4V 4EL (GB)**

(56) References cited:
**WO-A-93/14214          US-A- 5 135 860**
**US-A- 5 180 846**

**Description**

BACKGROUND OF THE INVENTION

1. Field of the Invention:

This invention relates to an improved process for the production of mixtures of glyoxylic acid and aminomethylphosphonic acid (AMPA), where glycolic acid and oxygen are reacted in an aqueous solution in the presence of AMPA and a catalyst. More specifically this invention relates to the use of a genetically-engineered microbial double-transformant which expresses two exogenous enzymes, glycolate oxidase (e.g., (S)-2-hydroxy-acid oxidase, EC 1.1.3.15) and a catalase (e.g., EC 1.11.1.6) which is not inhibited by AMPA (e.g., catalase T from *Saccharomyces cerevisiae)*, as catalyst.

2. Description of the Related Art:

Glycolate oxidase ("GO"), an enzyme commonly found in leafy green plants and mammalian cells, catalyzes the oxidation of glycolic acid to glyoxylic acid, with the concomitant production of hydrogen peroxide:

$$HOCH_2CO_2H + O_2 \rightarrow OCHCO_2H + H_2O_2$$

N. E. Tolbert et al., *J. Biol. Chem.,* Vol. 181, 905-914 (1949) first reported an enzyme, extracted from tobacco leaves, which catalyzed the oxidation of glycolic acid to formic acid and $CO_2$ via the intermediate formation of glyoxylic acid. The addition of certain compounds, such as ethylenediamine, limited the further oxidation of the intermediate glyoxylic acid. The oxidations were carried out at a pH of about 8, typically using glycolic acid concentrations of about 3-40 mM (millimolar). The optimum pH for the glycolate oxidation was reported to be 8.9. Oxalic acid (100 mM) was reported to inhibit the catalytic action of the glycolate oxidase. Similarly, K. E. Richardson and N. E. Tolbert, *J. Biol. Chem.*, Vol. 236, 1280-1284 (1961) showed that buffers containing tris(hydroxymethyl)aminomethane (TRIS) inhibited the formation of oxalic acid in the glycolate oxidase catalyzed oxidation of glycolic acid. C. O. Clagett, N. E. Tolbert and R. H. Burris, *J. Biol. Chem.,* Vol. 178, 977-987 (1949) reported that the optimum pH for the glycolate oxidase-catalyzed oxidation of glycolic acid with oxygen was about 7.8-8.6, and the optimum temperature was 35-40°C.

I. Zelitch and S. Ochoa, *J. Biol. Chem.,* Vol. 201, 707-718 (1953), and J. C. Robinson et al., *J. Biol. Chem.,* Vol. 237, 2001-2009 (1962), reported that the formation of formic acid and $CO_2$ in the spinach glycolate oxidase-catalyzed oxidation of glycolic acid resulted from the non-enzymatic reaction of $H_2O_2$ with glyoxylic acid. They observed that addition of catalase, an enzyme that catalyzes the decomposition of $H_2O_2$, greatly improved the yields of glyoxylic acid by suppressing the formation of formic acid and $CO_2$. The addition of FMN (flavin mononucleotide) was also found to greatly increase the stability of the glycolate oxidase.

N. A. Frigerio and H. A. Harbury, *J. Biol. Chem.,* Vol. 231, 135-157 (1958) have reported on the preparation and properties of glycolic acid oxidase isolated from spinach. The purified enzyme was found to be very unstable in solution; this instability was ascribed to the relatively weak binding of FMN to the enzyme active site, and to the dissociation of enzymatically active tetramers and/or octamers of the enzyme to enzymatically-inactive monomers and dimers, which irreversibly aggregate and precipitate. The addition of FMN to solutions of the enzyme greatly increased its stability, and high protein concentrations or high ionic strength maintained the enzyme as octamers or tetramers.

A process for the preparation of mixtures of glyoxylic acid and aminomethylphosphonic acid (AMPA) has been described in U.S. Patent 5,135,860. Glycolic acid and oxygen were reacted in an aqueous solution and in the presence of AMPA and two enzyme catalysts, the soluble spinach glycolate oxidase, and a soluble catalase (e.g., the soluble catalase from *Aspergillus niger).* This process demonstrated using both catalase (to destroy byproduct hydrogen peroxide responsible for formate production) and AMPA as an amine additive capable of forming oxidation-resistant N-substituted hemiaminal and/or imine complexes with glyoxylate (limiting its further oxidation). U.S. Patent 5,180,846 describes a process for the hydrogenation of these mixtures to produce N-(phosphonomethyl)glycine, a post-emergent phytotoxicant and herbicide. The present invention is viewed as a specific improvement of these processes.

SUMMARY OF THE INVENTION

The present invention involves the discovery of an inherent reversible inhibition of certain catalases when employed during the enzymatic oxidation of glycolic acid (and/or salt thereof) with oxygen in aqueous solution and in the presence of AMPA to produce mixtures of glyoxylic acid (and/or salt solutions thereof) and AMPA. The present invention, in general, takes advantage of using a genetically-engineered double-transformant microbial cell which expresses two exogenous enzymes, glycolate oxidase ((S)-2-hydroxy-acid oxidase, EC 1.1.3.15) (e.g., from spinach) and a catalase (EC 1.11.1.6), as catalyst. More specifically, the present invention provides for the expression of an exogenous enzyme catalase which is selected such as not to be inhibited by AMPA (e.g., catalase T from *Saccharomyces cerevisiae*). Such mixtures of glyoxylic acid and AMPA are useful for the preparation of N-(phosphonomethyl)glycine, a post-emergent herbicide.

Thus the present invention provides an improved process for preparing a mixture of glyoxylic acid and aminomethylphosphonic acid comprising the step of oxidizing glycolic acid with oxygen in an aqueous solution in the presence of aminomethylphosphonic acid and the enzymes glycolate oxidase and catalase wherein the improvement comprises using a microbial double-transformant cell catalyst that expresses two exogenous enzymes, glycolate oxidase and catalase.

BRIEF DESCRIPTION OF THE DRAWING AND BIOLOGICAL DEPOSITS

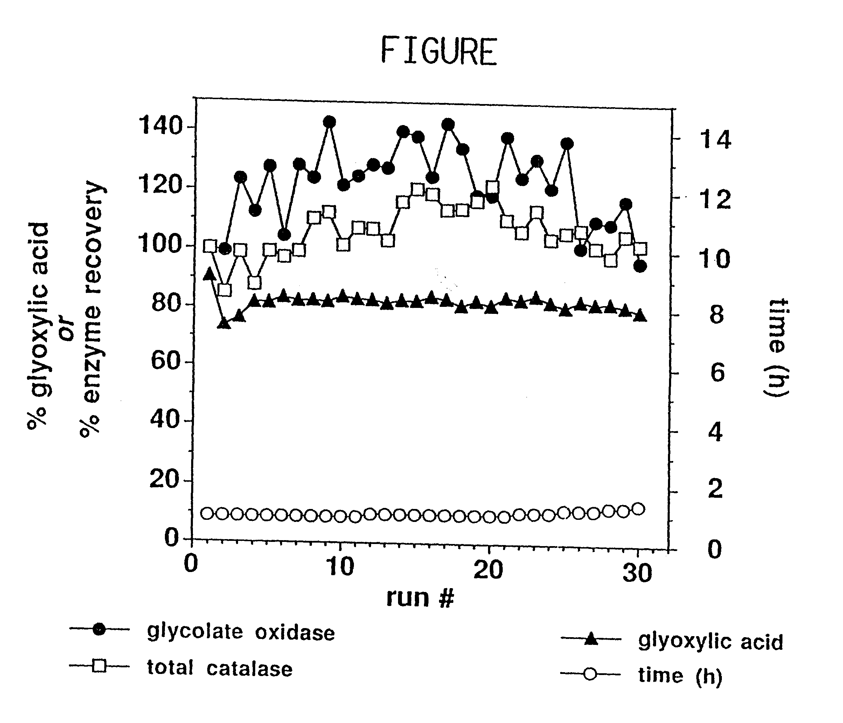

Figure 1 illustrates a plot of the reaction time, % yield of glyoxylic acid, and % recovery of permeabilized-cell glycolate oxidase and total catalase activities (based on their initial values after permeabilization) for each of thirty consecutive batch reactions performed using a *Hansenula polymorpha* double-transformant catalyst according to the present invention.

The following biological materials were deposited with the Agricultural Research Service Culture Collection (NRRL), International Depository Authority, 1815 N. University Street, Peoria, IL 61604, U.S.A. under the terms of the Budapest Treaty:

| Depositor's Designation | NRRL Accession # | Deposit Date |
| --- | --- | --- |
| *Hansenula polymorpha*, GO1 | Y-21065 | 30 March 1993 |
| *Pichia pastoris*, GS115-MSP10 | Y-21001 | 24 September 1992 |
| *Hansenula polymorpha*, GO2 | Y-21113 | 25 June 1993 |
| *Hansenula polymorpha*, GO3 | Y-21114 | 25 June 1993 |
| *Pichia pastoris*, MSP8.6 | Y-21187 | 1 February 1994 |

DESCRIPTION OF THE PREFERRED EMBODIMENTS

A related commonly-assigned patent application, U.S.S.N. 08/026,615, (WO 96/00793) describes the preparation of glyoxylic acid/AMPA mixtures using as catalyst one of several genetically-engineered microbial transformants which expresses both the enzyme glycolate oxidase from spinach and an endogenous catalase. When using either a *H. polymorpha* or *P. pastoris* microbial cell transformant as catalyst for the oxidation of glycolic acid/AMPA mixtures, the addition of a second source of catalase was found to be necessary for the production of high yields of glyoxylic acid. The accessible endogenous catalase activity of permeabilized cells of *H. polymorpha* or *P. pastoris* was less effective at decomposing byproduct hydrogen peroxide to water and oxygen in reaction mixtures containing AMPA than catalase from *A. nidulans* or *A. niger,* resulting in the production of significant quantities of formate (a product of the oxidation of glyoxylate by hydrogen peroxide). The addition of a soluble catalase from *A. niger* or *Saccharomyces cerevisiae* or permeabilized whole cells of *S. cerevisiae* as a supplemental catalase source resulted in marked improvements in glyoxylic acid production in reactions using either the *H. polymorpha* or *P. pastoris* transformant as catalyst.

The catalase from *Aspergillus niger, Saccharomyces cerevisiae* (catalase T), *Hansenula polymorpha, Pichia pastoris,* and bovine liver have now each been examined as catalyst for the decomposition of hydrogen peroxide generated during the oxidation of glycolic acid/AMPA mixtures. These catalases are all categorized as "cytostolic". A previously-unreported, reversible inhibition of the catalase from *H. polymorpha, P. pastoris,* and bovine liver by AMPA has been discovered. This inhibition occurs whether or not a soluble catalase, immobilized catalase, or catalase-containing whole cell catalyst is employed.

As an alternative to adding an additional source of inhibition-resistant catalase to glycolic acid/AMPA reaction mixtures which use as catalyst a microbial cell transformant whose endogenous catalase is inhibited by AMPA (e.g., *H. polymorpha* or *P. pastoris* transformants), the microbial transformant (which expresses the exogenous enzyme glycolate oxidase) has been reengineered to express an additional exogenous catalase, e.g., catalase T from *S. cerevisiae,* which is not inhibited by AMPA. Using this microbial cell "double" transformant as catalyst in the absence of an added catalase which is not inhibited by AMPA has resulted in a significant increase in glyoxylate yield and a reduction in formate production when compared to that obtained with a microbial cell "single" transformant (i.e., exogenous glycolate oxidase and an endogenous catalase) whose catalase is inhibited by AMPA.

A microbial cell double-transformant which contains both glycolate oxidase activity and an AMPA inhibition-resistant catalase activity offers several advantages as catalyst for the oxidation of glycolic acid/AMPA mixtures over the previously-employed combination of a microbial cell single-transformant with a soluble inhibition-resistant catalase: 1) the double-transformant is easily recovered for reuse from the reaction mixture at the conclusion of the reaction by centrifugation or filtration, whereas the soluble catalase employed in conjunction with the single-transformant is only recovered with great difficulty and loss of activity; 2) the inclusion of the inhibition-resistant catalase within the microbial cell catalyst results in a more stable catalase activity than that of soluble catalase, both for the number of catalyst turnovers obtained versus the soluble enzyme, as well as for recovered enzyme activity at the conclusion of a reaction; and 3) the double-transformants are stable to reaction conditions where oxygen is sparged into the reaction mixture to increase the rate of oxygen dissolution and reaction rate, where under similar reaction conditions the soluble catalase may be rapidly denatured, irreversibly losing its enzymatic activity.

A microbial cell catalyst which has been utilized in the present invention is a double-transformant of *Hansenula polymorpha* (a methylotrophic yeast) which expresses the glycolate oxidase enzyme from spinach, as well as the catalase T from *S. cerevisiae*. Several transformants of *H. polymorpha* having sufficient glycolate oxidase and catalase T activities have been prepared. For example, the CTT1 coding sequence for catalase T (originally from *S. cerevisiae*) as found in the plasmid pHCT-124 (Hansen and Roggenkamp, *Eur. J. Biochem.,* 184:173-179 (1989)) has been subcloned into a M13 vector (Ledeboer et al., *Nucleic Acids Res.,* 13, 3063 (1985) and Messing, Methods Enzymol; 101: 20-78 (1983)) and mutagenized by site-directed mutagenesis to obtain *Bgl*II restriction sites flanking the ATG start and the TAA stop codon, of the CTT1 gene. After insertion of the CTT1 gene and the ADH1/kanamycin resistance fusion into pRB the resulting vector was designated pRBCATT. It contained the CTT1 sequence inserted between the *FMD*-promoter and the *MOX*-terminator sequence as described for the GO expression plasmids. Transformation of *Hansenula polymorpha* GO1 identified by the accession number NRRL No. Y-21065 with pRBCATT and identification of strains coexpressing GO and catalase T is more fully described in commonly assigned U.S. Patent Application serial number U.S.S.N. 08/085,491, (WO 95/01443). When using plasmid pRBCATT, over seventy clones with varying expression levels in terms of enzyme activity per mg of protein of CTT1 in addition to the GO expression could be identified. Two individual strains of microbial double-transformant cells from this subcloning were designated respectively as GO2 and GO3 and identified by the accession numbers NRRL No. Y-21113 and Y-21114, respectively.

*H. polymorpha* double-transformants were typically prepared by first growing an inoculum of the double-transformant (500 mL) in a medium containing 0.7 g YNB (without ammonium sulfate), 0.5 g yeast extract, 2.5 g ammonium sulfate, 1 g gelatin hydrolysate (BBL), 1.5 g casamino acids (Difco), 0.3 g $K_2HPO_4$, 0.25 mg biotin, 1.25 mg thiamine, and 7.5 g glycerol for 24 h at 30°C and at pH 5.0 with 200 rpm agitation. The inoculum was then transferred to a 14 L fermenter charged with 10 L of 4.7 g/L spray dried corn steep (Roquette), 3.5 g/L ammonium sulfate, 0.36 mg/L biotin, 1.0 mg/L thiamine, and 30 g/L glycerol. The cells were grown for 15-20 h at 30°C with agitation of 500-550 rpm, aeration rate of 2-4 L/min air, and the pH was controlled at 5 using anhydrous ammonia. When growth-associated glycerol was depleted, glycolate oxidase and catalase T expression was induced by adding 10.0 g/L methanol to the fermenter, with supplemental addition of 0.67 g/L YNB (without amino acids or ammonium sulfate), 0.1 mg/L biotin, 0.5 mg/L thiamine, and 0.5 g/L glycerol. The methanol concentration was maintained at 2-10 g/L and the glycerol concentration was maintained at <1 g/L. Maximum glycolate oxidase and catalase T expression was reached at 20-30 h post induction.

At the conclusion of the fermentation, the cells were harvested by centrifugation and stored at -20°C. Glycolate oxidase, total catalase (*S. cerevisiae* catalase T plus endogenous *H. polymorpha* catalase), and catalase T activities present in extracts of *H. polymorpha* double-transformants ranged from 95 to 180 DCIP IU/gram wet cells for glycolate oxidase, 165,000 to 330,000 IU/gram wet cells for total catalase, and 90,000 to 175,000 IU/gram wet cells for catalase T.

An additional microbial cell catalyst which has been utilized in the present invention is a double-transformant of *Pichia pastoris* (a methylotrophic yeast) which expresses the glycolate oxidase enzyme from spinach as well as the catalase T from *S. cerevisiae*. Several transformants of *P. pastoris* having sufficient glycolate oxidase and catalase T activities have been prepared by inserting the DNA for glycolate oxidase and catalase T into an expression vector under the control of the alcohol oxidase (AOX) promoter. *P. pastoris* was transformed with this vector and a strain producing high levels of glycolate oxidase and catalase T was designated *P. pastoris* MSP8.6 and identified by the accession number NRRL No. Y-21187.

The *Pichia pastoris* double-transformant was prepared by first growing an inoculum (1.0 L) in a medium containing 3.0 g yeast extract, 3.0 g malt extract, 5.0 g peptone, and 10.0 g glycerol. The inoculum was then transferred to a 14 L fermenter charged with 9 L of 38.2 g/L $KH_2PO_4$, 9.7 g/L ammonium sulfate, 45 g/L glycerol, 10.53 g/L $MgSO_4 \cdot 7H_2O$, 0.81 g/L $CaSO_4 \cdot 2H_2O$, and 12.9 mL of a trace elements solution consisting of 6.0 g/L $CuSO_4 \cdot 5H_2O$, 0.8 g/L KI, 3.0 g/L $MnSO_4 \cdot H_2O$, 0.2 g/L $NaMoO_4 \cdot 2H_2O$, 0.02 g/L $H_3BO_3$, 0.50 g/L $CoCl_2 \cdot 6H_2O$, 20 g/L $ZnSO_4$, 5 mL/L $H_2SO_4$, 65 g/L $FeSO_4 \cdot 7H_2O$, and 0.2 g/L biotin. The cells were grown for 45 h at 30°C with agitation of 500-550 rpm, aeration rate of 5 L/min air, and the pH was controlled at 5.0 using ammonium hydroxide. When growth-associated glycerol was depleted, glycolate oxidase and catalase T expression was induced by adding to the fermenter 50 mL of an induction media prepared by mixing 1.5 L of methanol, 7.5 mL trace metals, and 7.5 mL of a 0.20 g/L biotin stock solution. The methanol concentration was maintained between 0.2-0.6% by the addition of additional induction media to the tank.

The cells were harvested 47 h post induction.

At the conclusion of the fermentation, the cells were harvested by centrifugation and stored at -20°C. Glycolate oxidase, total catalase *(S. cerevisiae* catalase T plus endogenous *P. pastoris* catalase), and catalase T activities present in extracts of *P. pastoris* double-transformants ranged from 218-254 DCIP IU/gram wet cells for glycolate oxidase, 233,000-252,000 IU/gram wet cells for total catalase, and 20,000-58,000 IU/gram wet cells for catalase T.

*H. polymorpha* and *P. pastoris* double-transformants required permeabilization prior to use as catalyst for the oxidation of glycolic acid to glyoxylic acid. A variety of known methods of permeabilization were useful for preparing cells with sufficient glycolate oxidase activity (see Felix, *Anal. Biochemistry,* Vol. 120, 211-234, (1982)). One method was performed by mixing a suspension of 10 wt % wet cells in a 0.1% (v/v) "Triton™" X-100/20 mM phosphate buffer (pH 7.0) for 15 min, then freezing in liquid nitrogen, thawing, and washing the permeabilized cells with 20 mM phosphate buffer (pH 7.0). A second method of permeabilization was performed by mixing a suspension of 10 wt % wet cells in 0.1% (w/v) benzalkonium chloride/50 mM phosphate buffer (pH 7.0) for 60 min, then washing the permeabilized cells with 50 mM phosphate buffer (pH 7.0). Once permeabilized, the amount of whole cell catalyst added to a reaction mixture was chosen so as to provide the desired concentrations of glycolate oxidase and catalase activities. Recoveries of permeabilized cell catalyst from reactions with glycolate oxidase and catalase activities of greater than 100% of their initial values were due to increased permeabilization of the whole-cell catalyst during the course of the reaction.

Many of the deficiencies of using soluble catalase in combination with a microbial single-transformant as catalysts for the production of glyoxylic acid/AMPA mixtures have been eliminated by employing a microbial cell double-transformant as catalyst. Recovery and reuse of the whole-cell catalyst was easily performed by centrifugation of the catalyst away from the reaction mixture and recycling it to fresh reaction mixture; in this manner, turnover numbers for glycolate oxidase of as high as $10^7$ have been obtained. The ability to sparge oxygen or an oxygen-containing gas through the reaction mixture without loss of catalase activity (as is observed for the soluble catalase used in combination with a microbial single-transformant) resulted in an improved recovery of catalase activity for catalyst recycle.

The glycolate oxidase activity (added as permeabilized microbial double-transformant) used in the reaction should be present in an effective concentration, usually a concentration of 0.01 to about 100 IU/mL, preferably about 0.1 to about 10 IU/mL. An IU (International Unit) is defined as the amount of enzyme that will catalyze the transformation of one micromole of substrate per minute. A procedure for the assay of the soluble enzyme is found in Zelitch and Ochoa, *J. Biol. Chem.,* Vol. 201, 707-718 (1953). A modification of this method is used to assay the glycolate oxidase activity of freshly-prepared or recycled microbial double-transformants. The double-transformants were assayed for glycolate oxidase activity by accurately weighing ca. 5-10 mg of the permeabilized wet cells into a 3 mL quartz cuvette containing a magnetic stirring bar and adding 2.0 mL of a solution which was 0.12 mM in 2,6-dichlorophenolindophenol (DCIP) and 80 mM in TRIS buffer (pH 8.3). The cuvette was capped with a rubber septum and the solution deoxygenated by bubbling with nitrogen for 5 min. To the cuvette was then added by syringe 0.040 mL of 1.0 M glycolic acid/1.0 M TRIS (pH 8.3) and the mixture was stirred while measuring the change in absorption with time at 605 nm ($\varepsilon = 22,000$).

The catalase activity resistant to inhibition by AMPA (e.g., catalase T from *S. cerevisiae,* added as permeabilized microbial double-transformant) used in the reaction should be present in an effective concentration of 50 to 100,000 IU/mL of reaction mixture, preferably 500 to 14,000 IU/mL. The total catalase activity *(S. cerevisiae* catalase T plus endogenous *H. polymorpha* or *P. pastoris* catalase) of permeabilized microbial double-transformants was assayed by accurately weighing ca. 2-5 mg of the permeabilized wet cells into a 3 mL quartz cuvette containing a magnetic stirring bar, then adding 2.0 mL of 16.7 mM phosphate buffer (pH 7.0) followed by 1.0 mL of 59 mM hydrogen peroxide in 16.7 mM phosphate buffer (pH 7.0) and stirring the suspension while measuring the change in absorption with time at 240 nm ($\varepsilon = 39.4$). The catalase T and endogenous catalase activities of microbial double-transformants were each determined by preparing extracts of the double-transformants and assaying the extracts as described above at pH 7.0 and at pH 4.0. Compared to their respective activities at pH 7.0, the *S. cerevisiae* catalase T retains 60% of its activity when at pH 4.0, while the endogenous *H. polymorpha* catalase retains only 7% of its activity at pH 4.0, and the endogenous *P. pastoris* catalase has no measurable activity at pH 4.0.

Glycolic acid (2-hydroxyacetic acid) is available commercially. In the present reaction its initial concentration may be in the range of 0.10 M to 2.0 M, preferably between 0.25 M and 1.0 M. It can be used as such or as a compatible salt thereof; that is, a salt that is water-soluble and whose cation does not interfere with the desired conversion of glycolic acid to glyoxylic acid, or the subsequent reaction of the glyoxylic acid product with the aminomethylphosphonic acid to form N-(phosphonomethyl)-glycine. Suitable and compatible salt-forming cationic groups are readily determined by trial. Representative of such salts are the alkali metal, alkaline earth metal, ammonium, substituted ammonium, phosphonium, and substituted phosphonium salts.

The conversion of glycolic acid to glyoxylic acid is conveniently and preferably conducted in aqueous media. Aminomethylphosphonic acid (AMPA), or a suitable salt thereof, is added to produce a molar ratio of AMPA/glycolic acid (starting amount) in the range of from 0.01/1.0 to 3.0/1.0, preferably from 0.25/1.0 to 1.05/1.0. After combining AMPA and glycolic acid in an aqueous solution, the pH of the resulting mixture is adjusted to a value between 6 and 10, preferably between 7.0 and 8.5. Within this pH range, the exact value may be adjusted to obtain the desired pH by adding any compatible, non-interfering base, including alkali metal hydroxides, carbonates, bicarbonates and phosphates. The pH of the reaction mixture decreases slightly as the reaction proceeds, so it is often useful to start the reaction near the high end of the maximum enzyme activity pH range, about 9.0-8.5, and allow it to drop during the reaction. The pH can optionally be maintained by the separate addition of a non-interfering inorganic or organic buffer, since enzyme activity varies with pH.

It is understood that glycolic and glyoxylic acid are highly dissociated in water, and at pH of between 7 and 10 are largely if not substantially entirely present as glycolate and glyoxylate ions. It will also be appreciated by those skilled in the art that glyoxylic acid (and its conjugate base, the glyoxylate anion) may also be present as the hydrate, e.g. $(HO)_2CHCOOH$ and/or as the hemiacetal, $HOOCCH(OH)OCH(OH)COOH$, which compositions and their anionic counterparts are equivalent to glyoxylic acid and its anion for the present purpose of being suitable reactants for N-(phosphonomethyl)glycine formation.

Flavin mononucleotide (FMN) is an optional added ingredient, used at a concentration of 0.0 to 2.0 mM, preferably 0.01 to 0.2 mM.

Oxygen ($O_2$), the oxidant for the conversion of the glycolic acid to glyoxylic acid, may be added as a gas to the reaction by agitation of the liquid at the gas-liquid interface either through a membrane permeable to oxygen or by sparging (bubbling) oxygen through the reaction mixture. It is believed that under most conditions, the reaction rate is at least partially controlled by the rate at which oxygen can be dissolved into the aqueous medium. Thus, although oxygen can be added to the reaction as air, it is preferred to use a relatively pure form of oxygen and even use elevated pressures. Although no upper limit of oxygen pressure is known, oxygen pressures up to 50 atmospheres may be used, and 15 atmospheres is preferred. Agitation is important to maintaining a high oxygen dissolution (hence reaction) rate. Any convenient form of agitation, such as stirring is useful.

The reaction temperature is an important variable, in that it affects reaction rate and the stability of the enzymes. A reaction temperature of 0°C to 40°C may be used, but the preferred reaction temperature range is from 5°C to 15°C. Operating in the preferred temperature range maximizes recovered enzyme activity at the end of the reaction. The temperature should not be so low that the aqueous solution starts to freeze. Temperature can be controlled by ordinary methods, such as, but not limited to, using a jacketed reaction vessel and passing liquid of the appropriate temperature through the jacket. The reaction vessel may be constructed of any material that is inert to the reaction ingredients.

Following the cessation of contacting the reaction solution with $O_2$, the permeabilized cells may be removed by decantation, filtration or centrifugation and reused. Flavin mononucleotide (FMN) may optionally be removed by contacting the solution with activated carbon. The solution containing glyoxylic acid and aminomethylphosphonic acid (which are believed to be in equilibrium with the corresponding hemiaminal and imine) is treated in accordance with any of the processes known to the art for producing N-(phosphonomethyl)glycine.

Catalytic hydrogenation is a preferred method for preparing N-(phosphonomethyl)glycine from a mixture containing glyoxylic acid and aminomethylphosphonic acid. Hydrogenation catalysts suitable for this purpose include (but are not limited to) the various platinum metals such as iridium, osmium, rhodium, ruthenium, platinum, and palladium; also various other transition metals such as cobalt, copper, nickel and zinc. The catalyst may be unsupported, for example, as Raney nickel or platinum oxide; or it may be supported, for example, as platinum on carbon, palladium on alumina, or nickel on kieselguhr. Palladium on carbon, nickel on kieselguhr, and Raney nickel are preferred. The hydrogenation can be performed at a pH of from 4 to 11, preferably from 5 to 10. The hydrogenation temperature and pressure can vary widely. The temperature is generally in the range of 0°C to 150°C, preferably from 20°C to 90°C, while the $H_2$ pressure is generally in the range of from about atmospheric to about 100 atmospheres, preferably from 1 to 10 atmospheres. N-(phosphonomethyl)glycine, useful as a post-emergent herbicide, may be recovered from the reduced solution, whatever the reducing method employed, by any of the recovery methods known to the art.

In the following examples, which serve to further illustrate the invention, the yields of glyoxylate, formate and oxalate and the recovered yield of glycolate are percentages based on the total amount of glycolic acid present at the beginning of the reaction. Analyses of reaction mixtures were performed using high pressure liquid chromatography: organic acid analyses were performed using a Bio-Rad HPX-87H™ column, and AMPA and N-(phosphonomethyl) glycine were analyzed using a Bio-Rad Aminex™ Glyphosate Analysis column.

## EXAMPLE 1

A 300 mL EZE-Seal™ stirred autoclave reactor equipped with Dispersimax™ Impeller (Autoclave Engineers) was charged with 100 mL of a solution containing glycolic acid (0.500 M), AMPA (0.375 M), isobutyric acid (0.100 M, HPLC internal standard), and FMN (0.01 mM) at pH 8.3 (adjusted with 50% NaOH), and the solution cooled to 5°C. To the reactor was then added 5.0 g of *Hansenula polymorpha* double-transformant GO2 (NRRL No. Y-21113) (357 IU glycolate oxidase and 600,000 IU total catalase (50% *Saccharomyces cerevisiae* catalase T, 50% *H. polymorpha* endogenous catalase)) which had been permeabilized by treatment with 0.1% benzalkonium chloride (Lonza Barquat MB-50). The pH of the resulting mixture was readjusted to 8.3 with 5% NaOH. This mixture was stirred at 750 rpm, which bubbled oxygen through the mixture via the action of the turbine impeller, and at 5°C under 40 psig of oxygen. The reaction was monitored by taking a 0.20 mL aliquot of the reaction mixture at regular intervals, filtering the aliquot using a Millipore ULTRAFREE® (Millipore Corp., McClean, VA, USA) 10,000 NMWL Filter Unit, and analyzing the filtrate by HPLC. After 1.5 h, the yields of glyoxylic acid, oxalic acid, and formic acid were 90.0%, 3.1%, and 1.8%, respectively, with 5.0% recovery of glycolic acid. The final activities of permeabilized-cell glycolate oxidase and total catalase were 146% and 113%, respectively, of their initial values after permeabilization.

The microbial cell catalyst was recovered from the reaction mixture described above by centrifugation. Without further treatment the cell pellet was mixed with 100 mL of fresh reaction mixture, and the reaction repeated. After 1.5 h, the yields of glyoxylic acid, oxalic acid, and formic acid were 84.3%, 4.9%, and 9.9%, respectively, with 1.7% recovery of glycolic acid. The final activities of permeabilized-cell glycolate oxidase and total catalase were 143% and 104% of their initial values after permeabilization. The percentage of total catalase for catalase T and *H. polymorpha* catalase in extracts of the recovered cells after two uses were 46% and 54%, respectively.

EXAMPLE 2 (COMPARATIVE)

The reaction described in Example 1 was repeated using 4.6 g of *Hansenula polymorpha* single-transformant G01 (NRRL No. Y-21065) (194 IU glycolate oxidase, 440,000 IU *H. polymorpha* endogenous catalase, and no catalase T) which had been permeabilized by treatment with 0.1% benzalkonium chloride (Lonza Barquat MB-50). After 1.5 h, the yields of glyoxylic acid, oxalic acid, and formic acid were 59.3%, 2.9%, and 34.4%, respectively, with 4.9% recovery of glycolic acid. The final activities of permeabilized-cell glycolate oxidase and catalase were 191% and 116% of their initial permeabilized values.

EXAMPLE 3 (COMPARATIVE)

The reaction described in Example 1 was repeated using 10.0 g of *Pichia pastoris* single-transformant GS115-MSP10 (NRRL No. Y-21001) (391 IU glycolate oxidase, 457,000 IU *P. pastoris* endogenous catalase, and no catalase T) which had been permeabilized by treatment with 0.1% "Triton" X-100/1 freeze-thaw. The reaction mixture was stirred at 1000 rpm and at 5°C under 120 psig of oxygen. After 1.0 h, the yields of glyoxylic acid, oxalic acid, and formic acid were 45.5%, 2.9%, and 37.1%, respectively, with 8.2% recovery of glycolic acid.

EXAMPLE 4

The reaction described in Example 1 was repeated at 15°C using 5.0 g of *Hansenula polymorpha* double-transformant GO2 (NRRL No. Y-21113) (402 IU glycolate oxidase and 588,000 IU total catalase (50% catalase T, 50% *H. polymorpha* catalase)) which had been permeabilized by treatment with 0.1% benzalkonium chloride (Lonza Barquat MB-50). After 1.0 h, the yields of glyoxylic acid, oxalic acid, and formic acid were 83.2%, 6.7%, and 9.5%, respectively, with 1.0% recovery of glycolic acid. The final activities of permeabilized-cell glycolate oxidase and total catalase were 132% and 127% of their initial permeabilized values.

EXAMPLE 5

The reaction described in Example 1 was repeated at 30°C using 5.0 g of *Hansenula polymorpha* double-transformant GO2 (NRRL No. Y-21113) (402 IU glycolate oxidase and 588,000 IU total catalase (50% catalase T, 50% *H. polymorpha* catalase)) which had been permeabilized by treatment with 0.1% benzalkonium chloride (Lonza Barquat MB-50). After 1.0 h, the yields of glyoxylic acid, oxalic acid, and formic acid were 65.5%, 5.7%, and 23.0%, respectively, with 5.5% recovery of glycolic acid. The final activities of permeabilized-cell glycolate oxidase and total catalase were 100% and 103% of their initial permeabilized values.

EXAMPLE 6

The reaction described in Example 1 was repeated using 10.0 g of *Hansenula polymorpha* double-transformant GO2 (NRRL No. Y-21113) (699 IU glycolate oxidase and 1,007,000 IU total catalase (50% catalase T, 50% *H. polymorpha* catalase)) which had been permeabilized by treatment with 0.1% benzalkonium chloride (Lonza Barquat MB-50). After 1.0 h, the yields of glyoxylic acid, oxalic acid, and formic acid were 89.4%, 4.4%, and 1.6%, respectively, with 2.5% recovery of glycolic acid. The final activities of permeabilized-cell glycolate oxidase and total catalase were 84% and 98% of their initial permeabilized values.

EXAMPLE 7

The reaction described in Example 1 was repeated using 2.0 g of *Hansenula polymorpha* double-transformant G02 (NRRL No. Y-21113) (140 IU glycolate oxidase and 201,000 IU total catalase (50% catalase T, 50% *H. polymorpha* catalase)) which had been permeabilized by treatment with 0.1% benzalkonium chloride (Lonza Barquat MB-50). After 1.75 h, the yields of glyoxylic acid, oxalic acid, and formic acid were 76.0%, 2.8%, and 14.0%, respectively, with 5.8% recovery of glycolic acid. The final activities of permeabilized-cell glycolate oxidase and total catalase were 155% and 126% of their initial permeabilized values.

EXAMPLE 8

The reaction described in Example 1 was repeated in the absence of added FMN at 70 psig* oxygen using 5.0 g of *Hansenula polymorpha* double-transformant GO2 (NRRL No. Y-21113) (383 IU glycolate oxidase and 602,000 IU total catalase (50% catalase T, 50% *H. polymorpha* catalase)) which had been permeabilized by treatment with 0.2% benzalkonium chloride (Lonza Barquat MB-50). After 1.75 h, the yields of glyoxylic acid, oxalic acid, and formic acid were 85.0%, 4.3%, and 7.4%, respectively, with 4.1% recovery of glycolic acid. The final activities of permeabilized-cell glycolate oxidase and total catalase were 104% and 99% of their initial permeabilized values.

EXAMPLE 9

The reaction described in Example 1 was repeated in the absence of added FMN at 90 psig* oxygen using 5.0 g of *Hansenula polymorpha* double-transformant G02 (NRRL No. Y-21113) (383 IU glycolate oxidase and 602,000 IU total catalase (50% catalase T, 50% *H. polymorpha* catalase)) which had been permeabilized by treatment with 0.2% benzalkonium chloride (Lonza Barquat MB-50). After 1.0 h, the yields of glyoxylic acid, oxalic acid, and formic acid were 87.2%, 6.8%, and 5.0%, respectively, with 2.3% recovery of glycolic acid. The final activities of permeabilized-cell glycolate oxidase and total catalase were 110% and 105% of their initial permeabilized values.

EXAMPLE 10

The reaction described in Example 1 was repeated in the absence of added FMN at 120 psig* oxygen using 5.0 g of *Hansenula polymorpha* double-transformant GO2 (NRRL No. Y-21113) (383 IU glycolate oxidase and 602,000 IU total catalase (50% catalase T, 50% *H. polymorpha* catalase)) which had been permeabilized by treatment with 0.2% benzalkonium chloride (Lonza Barquat MB-50). After 0.8 h, the yields of glyoxylic acid, oxalic acid, and formic acid were 91.3%, 5.6%, and 1.5%, respectively, with 3.6% recovery of glycolic acid. The final activities of permeabilized-cell glycolate oxidase and total catalase were 100% and 100% of their initial permeabilized values.

EXAMPLE 11

Into a 3 oz**. Fischer-Porter glass aerosol reaction vessel was placed a magnetic stirring bar and 10 mL of an aqueous solution containing glycolic acid (0.25 M), AMPA (0.25 M), FMN (0.01 mM), and isobutyric acid (HPLC internal standard, 0.10 M) at pH 8.3 (adjusted with 50% NaOH), and the solution cooled to 5°C. To the vessel was then added 0.50 g of *Hansenula polymorpha* double-transformant GO2 (NRRL No. Y-21113) (35.0 IU glycolate oxidase and 50,300 IU total catalase (50% catalase T, 50% *H. polymorpha* catalase)) which had been permeabilized by treatment with 0.1% benzalkonium chloride (Lonza Barquat MB-50), and the pH of the resulting mixture readjusted to 8.3 with 5% NaOH. The reaction vessel was sealed, then the vessel was flushed with oxygen by pressurizing to 70 psig* and venting to atmospheric pressure 5 times with stirring. The vessel was then pressurized to 70 psig* of oxygen and the mixture stirred at 5°C. Aliquots (0.10 mL) were removed by syringe through a sampling port (without loss of pressure in the vessel) at regular intervals, filtered using a Millipore "ULTRAFREE®-MC" 10,000 NMWL Filter Unit, and analyzed by HPLC to monitor the progress of the reaction. After 1 h, the yields of glyoxylic acid, oxalic acid, and formic acid were 89.7%, 9.1%, and 1.6%, respectively, and 0.8% glycolate remained. The final activities of permeabilized-cell glycolate oxidase and total catalase were 94% and 117%, respectively, of their initial values after permeabilization.

EXAMPLE 12

The reaction described in Example 11 was repeated using 0.50 M glycolic acid and 0.15 M AMPA. After 4.5 h, the yields of glyoxylic acid, oxalic acid, and formic acid were 87.6%, 6.4%, and 3.8%, respectively, and 2.0% glycolate

(* 1 psig = 6.893 kPa)
(** 1oz. = 2.835·10$^{-2}$kg)

remained. The final activities of permeabilized-cell glycolate oxidase and total catalase were 111% and 134%, respectively, of their initial values after permeabilization.

EXAMPLE 13

The reaction described in Example 11 was repeated using 0.50 M glycolic acid and 0.525 M AMPA. After 5.5 h, the yields of glyoxylic acid, oxalic acid, and formic acid were 85.3%, 6.7%, and 6.7%, respectively, and 1.5% glycolate remained. The final activities of permeabilized-cell glycolate oxidase and total catalase were 137% and 138%, respectively, of their initial values after permeabilization.

EXAMPLE 14

The reaction described in Example 11 was repeated using 0.50 M glycolic acid and 0.75 M AMPA. After 26 h, the yields of glyoxylic acid, oxalic acid, and formic acid were 50.9%, 0.9%, and 1.7%, respectively, and 43.1% glycolate remained. The final activities of permeabilized-cell glycolate oxidase and total catalase were 117% and 144%, respectively, of their initial values after permeabilization.

EXAMPLE 15 (COMPARATIVE)

The reaction described in Example 11 was repeated using 0.50 M glycolic acid, 0.375 M AMPA, and 0.47 g of *Hansenula polymorpha* single-transformant G01 (NRRL No. Y-21065) (10 IU glycolate oxidase, 22,100 IU *H. polymorpha* endogenous catalase, and no catalase T) which had been permeabilized by treatment with 0.1% "Triton" X-100/1 freeze-thaw. After 16 h, the yields of glyoxylic acid, oxalic acid, and formic acid were 57.6%, 2.6%, and 32.5%, respectively, with 8.9% recovery of glycolic acid. The final activities of permeabilized-cell glycolate oxidase and catalase were 60% and 378% of their initial permeabilized values.

EXAMPLE 16 (COMPARATIVE)

The reaction described in Example 11 was repeated using 0.50 M glycolic acid, 0.375 M AMPA, and 0.75 g of *Pichia pastoris* single-transformant GS115-MSP10 (NRRL No. Y-21001) (13.2 IU glycolate oxidase, 21,200 IU *P. pastoris* endogenous catalase, and no catalase T) which had been permeabilized by treatment with 0.1% "Triton" X-100/1 freeze-thaw. After 9.0 h, the yields of glyoxylic acid, oxalic acid, and formic acid were 30.5%, 10.7%, and 59.2%, respectively, with 0.8% recovery of glycolic acid.

EXAMPLE 17

A 300 mL EZE-Seal stirred autoclave reactor equipped with Dispersimax Impeller (Autoclave Engineers) was charged with 100 mL of a solution containing glycolic acid (0.500 M), AMPA (0.375 M), isobutyric acid (0.100 M, HPLC internal standard), and flavin mononucleotide (0.01 mM) at pH 8.3 (adjusted with 50% NaOH), and the solution cooled to 5°C. To the reactor was then added 5.0 g of *Hansenula polymorpha* double-transformant GO2 (NRRL No. Y-21113) (655 IU glycolate oxidase and 596,000 IU total catalase (49% catalase T, 51% *H. polymorpha* endogenous catalase)) which had been permeabilized by treatment with 0.2% benzalkonium chloride (Lonza Barquat MB-50). The pH of the resulting mixture was readjusted to 8.3 with 5% NaOH. This mixture was stirred at 700 rpm, which bubbled oxygen through the mixture via the action of the turbine impeller, and at 5°C under 40 psig* of oxygen. The reaction was monitored by taking a 0.20 mL aliquot of the reaction mixture at regular intervals, filtering the aliquot using a Millipore "ULTRAFREE®-MC" 10,000 NMWL Filter Unit, and analyzing the filtrate by HPLC. After 0.9 h, the yields of glyoxylic acid, oxalic acid, and formic acid were 90.6%, 7.4%, and 2.8%, respectively, with 2.0% recovery of glycolic acid.

The microbial cell catalyst was recovered from the reaction mixture described above by centrifugation. Without further treatment, the cell pellet was mixed with 100 mL of fresh reaction mixture and the reaction repeated. Thirty consecutive batch reactions were performed using this procedure with the same 5.0 g of *Hansenula polymorpha* double-transformant catalyst; the reaction time, % yield of glyoxylic acid, and % recovery of permeabilized-cell glycolate oxidase and total catalase activities (based on their initial values after permeabilization) for each of the thirty batch reactions are reported in Figure 1.

EXAMPLE 18

The reaction described in Example 17 was repeated using no added FMN and 7.1 g of *Hansenula polymorpha*

(* 1 psig = 6.893 kPa)

double-transformant GO3 (NRRL No. Y-21114) (465 IU glycolate oxidase and 770,000 IU total catalase (27% *S. cerevisiae* catalase T, 73% *H. polymorpha* endogenous catalase)) which had been permeabilized by treatment with 0.1% benzalkonium chloride (Lonza Barquat OJ-50). After 1.25 h, the yields of glyoxylic acid, oxalic acid, and formic acid were 86.8%, 3.8%, and 6.0%, respectively, with 3.5% recovery of glycolic acid. The final activities of permeabilized-cell glycolate oxidase and total catalase were 92% and 106% of their initial permeabilized values.

The microbial cell catalyst was separated from the reaction mixture described above by centrifugation. The supernatant (0.434 M glyoxylic acid/0.375 M AMPA) was filtered through a 0.2 micron cellulose acetate filter, then consecutively through an AMICON® YM30, YM10, and YM1 membrane filter (W. R. Grace & Co., New York, NY). The resulting filtrate was placed in a 300 mL EZE-Seal stirred autoclave reactor equipped with Dispersimax Impeller (Autoclave Engineers), along with 1.0 g of 5% palladium on carbon. The reactor was purged with nitrogen, then pressurized to 275 psig of hydrogen and the reaction mixture stirred at 1000 rpm and 27°C. After 6 h, the yield of N-(phosphonomethyl) glycine was 99.9% (based on AMPA, determined by HPLC).

## EXAMPLE 19

A 300 mL EZE-Seal stirred autoclave reactor equipped with Dispersimax Impeller (Autoclave Engineers) was charged with 100 mL of a solution containing glycolic acid (0.500 M), AMPA (0.375 M), isobutyric acid (0.100 M, HPLC internal standard), and FMN (0.01 mM) at pH 8.3 (adjusted with 50% NaOH), and the solution cooled to 5°C. To the reactor was then added 5.0 g of *Pichia Pastoris* double-transformant MSP8.6 (NRRL No. Y-21187) (234 IU glycolate oxidase and 543,000 IU total catalase (13% *S. cerevisiae* catalase T, 87% *P. pastoris* endogenous catalase)) which had been permeabilized by treatment with 0.1% benzalkonium chloride (Lonza Barquat MB-50). The pH of the resulting mixture was readjusted to 8.3 with 5% NaOH. This mixture was stirred at 750 rpm, which bubbled oxygen through the mixture via the action of the turbine impeller, and at 5°C under 70 psig of oxygen. The reaction was monitored by taking a 0.20 mL aliquot of the reaction mixture at regular intervals, filtering the aliquot using a Millipore "ULTRAFREE®-MC" 10,000 NMWL Filter Unit (Millipore Corp., Bedford, MA), and analyzing the filtrate by HPLC. After 1.0 h, the yields of glyoxylic acid, oxalic acid, and formic acid were 90.0%, 4.9%, and 4.4%, respectively, with 3.6% recovery of glycolic acid. The final activities of permeabilized-cell glycolate oxidase and total catalase were 125% and 160%, respectively, of their initial values after permeabilization.

## EXAMPLE 20 (COMPARATIVE)

The reaction described in Example 19 was repeated using 3.1 g of *Pichia pastoris* single-transformant GS115-MSP10 (NRRL No. Y-21001) (284 IU glycolate oxidase, 554,000 IU *P. pastoris* endogenous catalase, and no catalase T) which had been permeabilized by treatment with 0.1% benzalkonium chloride (Lonza Barquat MB-50). After 1.0 h, the yields of glyoxylic acid, oxalic acid, and formic acid were 78.8%, 3.2%, and 12.2%, respectively, with 4.8% recovery of glycolic acid. The final activities of permeabilized-cell glycolate oxidase and catalase were 101% and 139% of their initial permeabilized values.

## EXAMPLE 21

A 300 mL EZE-Seal stirred autoclave reactor equipped with Dispersimax Impeller (Autoclave Engineers) was charged with 100 mL of a solution containing glycolic acid (0.500 M), AMPA (0.375 M), isobutyric acid (0.100 M, HPLC internal standard), and FMN (0.01 mM) at pH 8.3 (adjusted with 50% NaOH), and the solution cooled to 5°C. To the reactor was then added 7.5 g of *Pichia Pastoris* double-transformant MSP-8.6 (NRRL No. Y-21187) (368 IU glycolate oxidase and 1,166,800 IU total catalase (12% *S. cerevisiae* catalase T, 88% *P. pastoris* endogenous catalase)) which had been permeabilized by treatment with 0.1% benzalkonium chloride (Lonza Barquat MB-50). The pH of the resulting mixture was readjusted to 8.3 with 5% NaOH. This mixture was stirred at 750 rpm, which bubbled oxygen through the mixture via the action of the turbine impeller, and at 5°C under 70 psig* of oxygen. The reaction was monitored by taking a 0.20 mL aliquot of the reaction mixture at regular intervals, filtering the aliquot using a Millipore "ULTRAFREE®-MC" 10,000 NMWL Filter Unit (Bedford, MA), and analyzing the filtrate by HPLC. After 1.0 h, the yields of glyoxylic acid, oxalic acid, and formic acid were 92.4%, 7.8%, and 0.3%, respectively, with 1.8% recovery of glycolic acid. The final activities of permeabilized-cell glycolate oxidase and total catalase were 118% and 91%, respectively, of their initial values after permeabilization.

The microbial cell catalyst was recovered from the reaction mixture described above by centrifugation. Without further treatment the cell pellet was mixed with 100 mL of fresh reaction mixture, and the reaction repeated. After 1.0 h, the yields of glyoxylic acid, oxalic acid, and formic acid were 85.0%, 5.2%, and 10.0%, respectively, with 2.6% recovery of glycolic acid. The final activities of permeabilized-cell glycolate oxidase and total catalase were 116% and

(* 1 psig = 6.893 kPa)

65% of their initial values after permeabilization. The percentage of total catalase for catalase T and *H. polymorpha* catalase in extracts of the recovered cells after two uses were 8.0% and 92%, respectively.

<u>EXAMPLE 22 (COMPARATIVE)</u>

The reaction described in Example 19 was repeated using 5.0 g of *Pichia pastoris* single-transformant GS115-MSPIO (NRRL No. Y-21001) (400 IU glycolate oxidase, 1,720,000 IU *P. pastoris* endogenous catalase, and no catalase T) which had been permeabilized by treatment with 0.1% benzalkonium chloride (Lonza Barquat MB-50). After 1.0 h, the yields of glyoxylic acid, oxalic acid, and formic acid were 78.1%, 7.2%, and 11.7%, respectively, with 2.7% recovery of glycolic acid. The final activities of permeabilized-cell glycolate oxidase and catalase were 105% and 87% of their initial permeabilized values.

The microbial cell catalyst was recovered from the reaction mixture described above by centrifugation. Without further treatment the cell pellet was mixed with 100 mL of fresh reaction mixture, and the reaction repeated. After 1.5 h, the yields of glyoxylic acid, oxalic acid, and formic acid were 52.8%, 4.0%, and 39.1%, respectively, with 3.0% recovery of glycolic acid. The final activities of permeabilized-cell glycolate oxidase and catalase were 107% and 44% of their initial values after permeabilization.

**Claims**

1. A process for preparing a mixture of glyoxylic acid and aminomethylphosphonic acid comprising the step of oxidizing glycolic acid with oxygen in a aqueous solution in the presence of aminomethylphosphonic acid and the enzymes glycolate oxidase and catalase characterised in that a microbial double-transformant cell catalyst that expresses the exogenous enzymes glycolate oxidase and catalase is used, the exogenous enzyme catalase being selected such as not to be inhibited by aminomethylphosphonic acid.

2. The process of Claim 1 wherein the exogenous enzyme catalase is derived from *Saccharomyces cerevisiae* or *Aspergillus niger.*

3. The process of Claim 1 wherein said microbial double-transformant cell is a methylotrophic yeast.

4. The process of Claim 3 wherein said methylotrophic yeast is selected from *Hansenula* and *Pichia sp.*

5. The process of Claim 1 wherein said microbial double-transformant cell is *Hansenula polymorpha* (NRRL Nos.: Y-21113 or Y-21114).

6. The process of Claim 1 wherein said microbial double-transformant cell is *Pichia pastoris* (NRRL No.: Y-21187).

**Patentansprüche**

1. Verfahren zur Herstellung eines Gemisches aus Glyoxylsäure und Aminomethylphosphonsäure, umfassend den Schritt der Oxidation von Glycolsäure mit Sauerstoff in einer wäßrigen Lösung in Gegenwart von Aminomethylphosphonsäure und den Enzymen Glycolatoxidase und Katalase, dadurch gekennzeichnet, daß ein mikrobieller Katalysator doppelt transformierter Zellen, der die exogenen Enzyme Glykolatoxidase und Katalase exprimiert, verwendet wird, wobei das exogene Enzym Katalase so gewählt wird, daß es durch die Aminomethylphosphonsäure nicht gehemmt wird.

2. Verfahren nach Anspruch 1, bei dem das exogene Enzym Katalase von Saccharomyces cerevisiae oder Aspergillus niger stammt.

3. Verfahren nach Anspruch 1, bei dem die mikrobielle doppelt transformierte Zelle eine methylotrophe Hefe ist.

4. Verfahren nach Anspruch 3, bei dem die methylotrophe Hefe ausgewählt wird aus Hansenula und Pichia sp.

5. Verfahren nach Anspruch 1, bei dem die mikrobielle doppelt transformierte Zelle Hansenula polymorpha (NRRL-Nrn. Y-21113 oder Y-21114) ist.

**6.** Verfahren nach Anspruch 1, bei dem die mikrobielle doppelt transformierte Zelle Pichia pastoris (NRRL-Nr. Y-21187) ist.

**Revendications**

**1.** Un procédé pour préparer un mélange d'acide glyoxylique et d'acide aminométhylphosphonique comprenant l'étape d'oxydation de l'acide glycolique par l'oxygène dans une solution aqueuse en présence d'acide aminométhylphosphonique et des enzymes glycolate-oxydase et catalase, caractérisé en ce qu'on utilise une cellule de double transformant microbien comme catalyseur qui exprime les enzymes glycolate-oxydase et catalase exogènes, l'enzyme catalase exogène étant choisie de manière à ne pas être inhibée par l'acide aminométhylphosphonique.

**2.** Le procédé de la revendication 1, dans lequel l'enzyme catalase exogène est dérivée de *Saccharomyces cerevisiae* ou de *Aspergillus niger.*

**3.** Le procédé de la revendication 1, dans lequel ladite cellule de double transformant microbien est une levure méthylotrophique.

**4.** Le procédé de la revendication 3, dans lequel ladite levure méthylotrophique est choisie parmi *Hansenula* et *Pichia sp.*

**5.** Le procédé de la revendication 1, dans lequel ladite cellule de double transformant microbien est *Hansenula polymorpha* (NRRL N° : Y-21113 ou Y-21114).

**6.** Le procédé de la revendication 1, dans lequel ladite cellule de double transformant microbien est *Pichia pastoris* (NRRL N° : Y-21187).

## FIGURE